# EUROPEAN PATENT SPECIFICATION

(11) **EP 0 343 114 B1**
(45) Date of publication and mention of the grant of the patent: **06.10.1993**
(21) Application number: 89810377.5
(22) Date of filing: 22.05.1989
(51) Int. Cl.: A61M 1/00

(54) **Transcutaneous device**
Transkutanvorrichtung
Dispositif transdermique

(30) Priority: 20.05.1988 JP 123267/88
(43) Date of publication of application: 23.11.1989
(73) Proprietor: ASAHI KOGAKU KOGYO KABUSHIKI KAISHA, Tokyo 174 (JP)
(72) Inventor: Koshikawa, Shozo, Tokyo (JP); Kitaoka, Tateki, Chiba-ken (JP); Nakabayashi, Nobuo, Chiba-ken (JP); Ichitsuka, Takeshi c/o Asahi Kogaku Kogyo K.K., Tokyo (JP); Ojima, Satoshi c/o Asahi Kogaku Kogyo K.K., Tokyo (JP)
(74) Representative: Moinas, Michel

(56) References cited:
- EP-A- 0 120 689
- FR-A- 2 310 322

## Description

The present invention relates to a transcutaneous device. More particularly, the present invention relates to a transcutaneous device having especially a good compatibility with and good adhesion to the subcutaneous tissue which is useful to fix and retain medical equipments embedded into a patient's body, for example, catheter and other communication means, in the surface of the patient's body.

Hitherto, there has been frequently carried out to embed tube means such as catheter or intubation into a living body or human body, particularly patient's body. An end part of the tube means is projected from the body surface, and is used as an input or output mean in a clinical field. For example, it can be used to take a sample of blood or biological fluid to obtain biological informations. Further, it can be used to supply a solution of a drug or drugs. It can be also used to conduct dialysis of blood, i.e., withdrawal of the old blood and supply of the fresh blood in an extracorporeal dialysis operation in the treatment of the kidney diseases. The prior art catheter and intubation are generally made of, for instance, silicone rubbers or fluorine plastics.

However, the conventional catheter and intubation made of silicone rubbers or fluorine resins have some drawbacks. Since a satisfactory adhesion of the catheter or intubation to the skin cannot be obtained because of properties of the tube material used, if the catheter or intubation is retained in the patient's body for an extended length of time, it can cause cutis defects such as so-called "down growth" of the skin. Also, it can sometimes cause a dermatitis or cutaneous inflammation and finally peritonitis and other inflammations due to invasion of bacteria or virus through a gap between the tube and cutaneous tissue into the body.

To remove said drawbacks and also improve an adhesion of the tube means to the skin, there has been suggested to use a transcutaneous device as a fixing mean in combination with the tube means. The transcutaneous device is made of hydroxyapatite which is a typical example of calcium phosphate ceramics having an excellent biocompatibility. The transcutaneous device of hydroxypatite can exhibit a satisfactorily good contactability with the soft subcutaneous tissue, since a surface of the device to be contacted with the soft tissue has a dense structure of apatite. However, the transcutaneous device still suffers from drawbacks that it has only a very weak adhesion to the soft tissue and thus it has a less fixability in the patient's body.

EP-A-0 120 689 is describing a transcutaneously implantable element in which at least a portion thereof in contact with the cutaneous tissue of a living body is composed of a ceramic material and which comprises a through hole for mechanically connecting the interior and exterior of the living body to each other.

Said element may be constituted of a head, that portion projecting from the skin surface, and a bottom, that portion embedded into the subcutaneous tissue of the patient. Head and bottom may be distinct parts or integrally combined with each other and are composed of the same ceramic material. It it should be noted that, in order to prevent any undesirable bacterial infection, the head is preferably provided with a internal cylinder made of a metal or a synthetic resin and equipped with a membrane filter.

Therefore, it has been desired to provide an improved transcutaneous device having no drawback encountered in the conventional transcutaneous device.

More particularly, a need exists for an improved transcutaneous device which has an increased adhesion property to the tissue within the living body, can prevent invasion of bacteria or virus into the body, and can be safely and stably retained in the body for a long period. Also, another need exists for an improved transcutaneous device which has a good fixing or anchoring effect enough to withstand an external physical pressure applied thereto, even if such pressure is applied at an initial stage of embedding of the same.

According to the present invention as disclosed in claim 1, there is provided a transcutaneous device for retaining a medical equipment such as catheter, intubation and other tube means for communicating an inside of the patient's body with an outside thereof, for instance, in the patient's body. Said transcutaneous device has a through-hole for receiving said medical equipment and comprises a portion adapted to be embedded into a subcutaneous tissue of the patient which is composed of porous ceramic material. Portions other than the portion to be contacted with said subcutaneous tissue are composed of a dense ceramic material.

The transcutaneous device of the present invention, comprises a composite structure of the porous ceramic material and dense ceramic material. In other words, in the transdermal element, portions other than the portion to be contacted with the subcutaneous tissue, for example, a wall portion of the through-hole and a portion to be projected from the skin surface, is composed of a dense ceramic material.

As described hereinafter, the transcutaneous device of the present invention can provide many remarkable advantages. First, based on an anchoring effect which principally relies upon pores of the porous ceramic material, the transcutaneous device can possess an excellent adhesion power to the living tissue, can prevent invasion of bacteria or the like, and can be safely and stably retained in the body for a long time, in addition to its own good biocompatibility. Second, in cases that an inner wall of the through-hole is constituted from a dense material and particularly that with a high mechanical strength, the transdermal element can exhibit an excellent fixability in the body and also can withstand an external physical pressure applied thereto at an initial stage of embedding of the same in the body.

Accordingly, the transcutaneous device of the present invention is particularly useful in inserting and safely and stably retaining catheter, intubation and other medical equipments for a long time in the subcutaneous tissue of the body. The transcutaneous device can be thus widely used in clinical applications with much advantages. Typical applications which are considered to be appropriate in the practice of the present invention include a renal dialysis in the treatment of the kidney trouble, a peritoneal dialysis, and determination of information of the living body such as determination of a blood sugar value for the diabetes patients, although the present invention is not limited to these applications.
Fig. 1 is a cross-sectional view illustrating embedding of the transcutaneous device according to another preferred embodiment of the present invention into a subcutaneous tissue of the body, and
Fig. 2 is a photomicrograph (50 x magnification) showing embedding of the transcutaneous device of the appended Example 1 into the subcutaneous tissue of the back region of the tested rat.

The transcutaneous device of the present invention, as described above, is composed of a porous ceramic material, at least, in a portion thereof to be contacted with the subcutaneous tissue. The element is partially composed of the porous ceramic material and only the portion contacted with the subcutaneous tissue when embedded into the body is composed of the porous ceramic material.

The porous ceramic material used in the present invention can be selected from a plurarity of well-known porous ceramics. Selection of such porous ceramic material is not limited, insofar as the selected material has no toxicity in vivo, namely, it does not adversely affect on a patient's body. Useful porous ceramic material includes, for example, calcium phosphate ceramics, alumina ceramics, zirconia ceramics and a composite of these ceramics. Especially useful ceramic material is calcium phosphates such as hydroxyapatite, because these materials have a high biocompatibility.

The porous ceramic material used has preferably a porosity of about 20 to 70%, more preferably a porosity of about 30 to 60%. The porosity of less than 20% must be avoided, because it does not ensure a desired anchoring effect. Also, the porosity of more than 70 % must be avoided, because it causes reduction of the mechanical strength to a level not enough to use in practical applications.

In addition to the porosity, the porous ceramic material has preferably a pore size or diameter of about 2 to 2000µm, more preferably a pore size of about 10 to 1000µm. The pore size of less than 2µm causes a drawback that the soft tissue can be grown into pores of the ceramic material with difficulty, while the pore size of more than 2000µm causes another drawback that only a less anchoring effect can be obtained as a result of reduction of the number of pores in the ceramic material.

The transcutaneous device of the present invention has a through-hole for receiving medical equipments such as catheter, intubation or the like. The size or diameter of the through-hole may be widely varied depending upon various factors such as specific applications and sizes of the medical equipments inserted and received in the hole. Similarly, the size and configuration of the transcutaneous device may be widely changed depending upon various factors such as specific sites to be embedded. Preferably, the configuration of the transcutaneous device is such that the element has an increased cross section in the direction of depth of the skin, when the element is embedded in the skin. The increased cross section of the transcutaneous device is particularly effective to more improve the resulting fixing effect.

The transcutaneous device according to the present invention will be described with reference to the accompanying drawings.

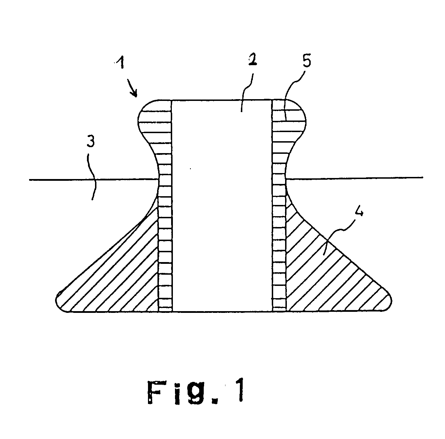

To prevent permeation of bacteria, the transcuraneous device 1 of Fig. 1 is composed of a porous shaped body 4 and a dense shaped body 5 and is provided with a through-hole 2. The through-hole 2 has a diameter enouth to receive the medical equipment therein. It will be appreciated that to improve an adhesion to the subcutaneous tissue 3, only the portions to be contacted with the tissue are made of the porous ceramic material. In this embodiment, the porous ceramic material may have open pores or closed pores.

Any dense ceramic material can be used in the formation of the dense shaped body 5, insofar as it has no toxicity to the patient's body. As dense ceramic materials, calcium phosphate ceramics are most preferably used. It may be of the same or of different nature from the porous ceramic material constituing the porous shaped body.

The transcutaneous device of the type illustrated in Fig. 1 can be prepared by a process comprising separately making the portion of the porous ceramic material and the portion of the dense ceramic material, optionally calcinating the resulting porous and dense shaped bodies, fitting and combining said bodies and sintering the resulting combination of said bodies to yield an integral structure of said bodies upon shrink fitting of said bodies.

The porous ceramic portion of the porous shaped body 4 can be produced by using conventional methods. For example, the porous ceramic material can be produced by preparing spherical ceramic particles and sintering said particles while they are contacted with each other. Further, the porous material can be produced by adding a foaming agent to a slurry of ceramic and then foaming and drying the mixture. Suitable foaming agent is a substance capable of forming foams in the slurry, i.e., for example, hydrogen peroxide and egg albumen. Further, the porous material can be also produced by adding a thermally removable substance to ceramic powders, molding the mixture to desired shape and sintering the molded product. The thermally removable substance useful in this method includes a sublimable solid substance such as adamantane or the like or a vaporizable organic substance which can be removed through vaporization from the molded product during sintering thereof such as organic resin beads, organic fibers or the like. Furthermore, the porous ceramic material can be produced by impregnating a slurry of ceramics into a combustible polymeric sponge or foam having a three-dimensional network and then sintering the impregnated and dried sponge. Of course, other production methods may be used, if desired.

The dense ceramic portion of the dense shaped body 5 can be produced in a manner similar to that described above.

After formation of the porous and dense shaped bodies, a through-hole is bored in the porous shaped body. Formation of the through-hole can be attained, for example, by machining or cutting. Alternatively, if desired, it may be carried out at the same time with shaping of the porous ceramic portion. The dense shaped body has a configuration suited to insert it into the through-hole of the porous shaped body. Such configuration can be produced during shaped of the dense ceramic portion or otherwise by machining or cutting after formation of the dense shaped body having a configuration roughly similar to that of the final product.

The porous shaped body with the through-hole and the dense shaped body are then fitted by inserting at least a part of the latter into the through-hole of the former. However, for stabilization and other purposes, the shaped bodies may be optionally fired at an appropriate calcination temperature, before they are fitted. Further, as described hereinafter, the dense shaped body may be pre-sintered to ensure an integral and strong bond between the bodies, if a shrinkage factor of the porous body is equal to or lower than that of the dense body.

The configuration of the through-hole of the porous body and that of the part of the dense body to be inserted into the through-hole of the porous body are not specially limited and therefore may be widely varied depending on various factors. However, to obtain a complete fitting of the bodies, a size or diameter of the through-hole of the porous body and a size or diameter of the dense body are preferably selected so that said dense body can be easily inserted into the through-hole of said porous body, but after fitting and sintering, an outer peripheral surface of said dense body can intimately contact and integrally bond with an inner peripheral surface of said porous body or surface of the through-hole as a result of excessive shrinkage of said porous body compared with said dense body. In other words, when the dense body is inserted into the through-hole of the porous body, a small gap should be generated between these bodies, but such gap is no longer found after completion of sintering. The size of the gap generated must be determined in each of the production of transcutaneous devices, because it can vary to a large extent depending upon various factors such as specific diameter of the through-hole, specific material of the ceramics used and specific sintering temperature. Note, assuming that both of the porous body and dense body are made from hydroxyapatite, after sintering at 1200°C, the porous body and dense body will show a shrinkage factor of about 66% and about 80%, respectively.

In the practice of the illustrated embodiment according to the present invention, the porous and dense bodies are preferably produced with such sizes that there is generated a gap of about 10 to 500µm between an inner surface of the through-hole of the porous body and an outer peripheral surface of the dense body. These bodies are simultaneously sintered to form an integral structure, after they were fitted.

The above-described production of the porous and dense bodies is particularly available to cases in which a shrinkage factor of the porous body higher than that of the dense body. On the other hand, for other cases in which a shrinkage factor of the porous body is equal to or lower than that of the dense body, it is suggested to previously sinter or pre-sinter only the dense body. This pre-sintering is considered to be effective to ensure that at the stage of sintering, the porous body excessively shrink in comparison with the dense body and thus the through-hole of the former can have a diameter of about 10 to 500µm smaller than the outer diameter of the latter. After pre-sintering, the porous and dense bodies are fitted and sintered.

Sintering of the fitted bodies is preferably carried out at an elevated temperature slightly higher than the sintering temperature generally applied to the conventional sintering processes for ceramic articles to yield a transcutaneous device of the integral structure. As typical examples of sintering, when both of the porous and dense bodies are made from calcium phosphate ceramics, sintering can be advantageously carried out at a temperature of about 1000 to 1250°C. Further, when both of the porous and dense bodies are made from zirconia ceramics, sintering can be advantageously carried out at a temperature of about 1400 to 1600°C. Furthermore, when the porous body and dense body are made of hydroxyapatite and alumina, respectively, sintering can be advantageously carried out at a temperature of about 1200 to 1400°C.

The present invention will be further described by reference to the following example to which the present invention is not restricted.

### Example 1 :

This example is included to explain the production and use of the transcutaneous device of the present invention illustrated in Fig. 1.

A dried porous material of hydroxyapatite was prepared as follows: Amorphous hydroxyapatite powders (particle size of 0.1 to 1µm) and spherical hydroxyapatite powders (particle size of 2 to 20µm) were blended. To the resulting blend, added are water and an aqueous hydrogen peroxide as a foaming agent. The mixture was kneaded and then foamed and dried in a constant temperature drying apparatus.

The dried porous material thus obtained was fabricated on a machining tool to obtain a porous body having a configuration illustrated in Fig. 1. The porous body was sintered at a temperature of 1200°C to produce a desired trancutaneous device. The trancutaneous device indicated that it has a porosity of 30% and a pore size of 5 to 1000µm.

In a separate step, a dried porous material of hydroxyapatite was prepared as follows : Amorphous hydroxyapatite powders (particle size of 0.1 to 1µm) were molded on a dry-type rubber press to obtain a green compact. The green compact was then machined to obtain a dense body with a through-hole. The configuration of the dense body was identical with that of the body 5 of Fig. 1.

This dried porous material was machined to obtain a porous body which is identical with the body 4 of Fig. 1. The dense body 5 was inserted into the porous body 4 and then sintered at a temperature of 1200°C. A desired transcutaneous device with the composite structure of Fig. 1 was produced upon shrink fitting of the bodies 4 and 5. The transcutaneous device indicated that the porous body portion thereof has a porosity of 30% and a pore size of 5 to 1000µm.

To ascertain excellent effects of the transcutaneous device produced in said process, a part of the transcutaneous device was implanted in a subcutaneous tissue of the back site of the male rats (weight of 200 to 250g), while the remainder of the transcutaneous device was exposed from the rat body. Adhesion and reaction between the transdermal element and the subcutaneous tissue was observed.

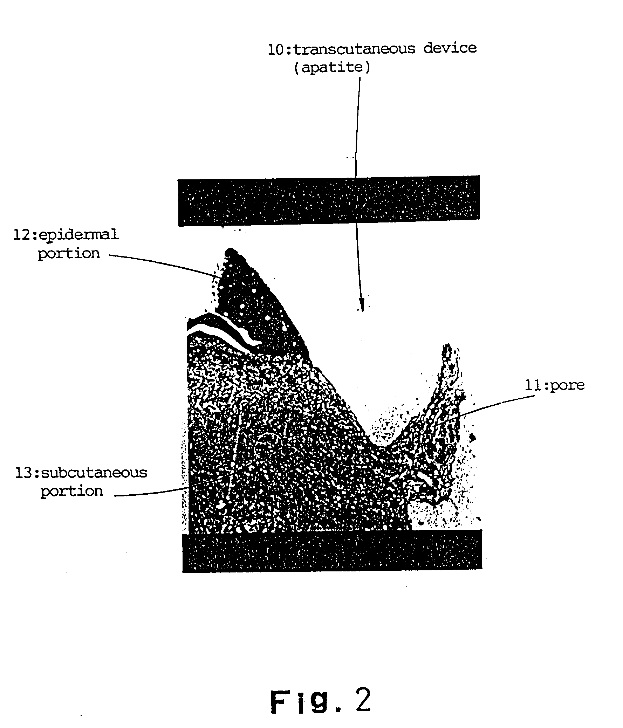

After 20 weeks, the tested transcutaneous device with the tissue was removed from the rat body to make a tissue preparation. The tissue preparation indicated that a good adhesion could be obtained between the transcutaneous device and the surrounding tissue. In addition to his visual inspection, a photomicrograph (50 x magnification) of the same tissue preparation showed a strong anchoring effect (see, Fig. 2). As shown in Fig. 2, a subcutaneous tissue 13 below an epidermis 12 could grow into pores 11 of the transcutaneous device 10. No down growth was observed in the epidermic region. Further, since the subcutaneous tissue has a good adhesivity to the transcutaneous device, bacteria infection or the like into the tissue could be prevented. As a result, no suppurative lesion was observed in the subcutaneous tissue surrounding the transcutaneous devce. Furthermore, microscopic inspection evidenced that there is no clumping image of the inflammatory cells.

## Claims

1. A transcutaneous device (1) for retaining a medical equipment in a patient's body, which has a through-hole (2) for receiving said medical equipment , said device comprising a portion (4) adapted to be embedded into a subcutaneous tissue (3) of the patient and which is composed of porous ceramic material, characterized in that portions (5) other than the portion to be contacted with said subcutaneous tissue (3) are composed of a dense ceramic material.

2. A transcutaneous device according to claim 1 wherein said porous ceramic material is a member selected from the group consisting of calcium phosphate ceramics, alumina ceramics, zirconia ceramics and a composite thereof.

3. A transcutaneous device according to claim 2 wherein said porous ceramic material is hydroxyapatite.

4. A transcutaneous device according to claim 1 wherein said porous ceramic material has a porosity of 20 to 70% and a pore size of 2 to 2000 micrometers.

5. A transcutaneous device according to claim 1 wherein said dense ceramic material is the same nature or of a different nature from said porous ceramic material.

6. A transcutaneous device according to claim 5 wherein both of said porous and dense ceramic materials used are hydroxyapatite.

7. A transcutaneous device according to claim 1 which is prepared by a process comprising separately molding the portion of the porous (4) ceramic material and the portion of the dense (5) ceramic material, optionally calcinating said portion, fitting and combining said portions and sintering a combination of said portions to yield an integral structure of said portions upon shrink fitting of said portions.

8. A transcutaneous device according to claim 7 wherein sintering is carried out at a temperature of 1000 to 1250xC when both of said porous (4) and dense (5) ceramic materials used are calcium phosphate ceramics.

9. A transcutaneous device according to claim 7 wherein sintering is carried out at a temperature of 1400 to 1600xC when both of said porous (4) and dense (5) ceramic materials used are zirconia ceramics.

10. A transcutaneous device according to claim 7 wherein sintering is carried out at a temperature of 1200 to 1400xC when said porous (4) ceramic material used is hydroxyapatite and dense (5) ceramic material used alumina.

11. A transcutaneous device according to anyone of claims 1 to 10 which is used to retain a catheter in the patients body.

## Patentansprüche

1. Transkutanvorrichtung (1) zum Festhalten einer medizinischen Einrichtung im Körper eines Patienten, enthaltend ein Durchgangsloch (2) zur Aufnahme der genannten medizinischen Einrichtung, wobei die Vorrichtung einen Teil (4) aufweist, welcher zum Einsetzen in ein subkutanes Gewebe (3) des Patienten ausgebildet ist und welcher aus porösem keramischem Material zusammengesetzt ist, dadurch gekennzeichnet, daß der Teil (5), welcher ein anderer ist als der in Kontakt mit dem subkutanen Gewebe (3) stehende Teil, aus dichtem keramischem Material bestehet.

2. Transkutanvorrichtung nach Anspruch 1, wobei das poröse keramische Material rein ausgewähltes Mitglied einer Gruppe ist, enthaltend Kalziumphosphat-Keramik, Aluminiumoxid-Keramik, Zirkoniumkeramik oder Zusammensetzungen von diesen.

3. Transkutanvorrichtung nach Anspruch 2, wobei das genannte poröse Keramikmaterial Hydroxylapatit ist.

4. Transkutanvorrichtung nach Anspruch 1, wobei das genannte poröse Keramikmaterial eine Porosität von 20 bis 70 % und einer Porengröße von 2 bis 2000 µm aufweist.

5. Transkutanvorrichtung nach Anspruch 1, wobei das genannte dichte keramische Material von der gleichen Art oder von einer unterschiedlichen Art ist wie die des porösen keramischen Materials.

6. Transkutanvorrichtung nach Anspruch 5, wobei sowohl das genannte poröse als auch das eingesetzte dichte keramische Material aus Hydroxylapatit bestehen.

7. Transkutanvorrichtung nach Anspruch 1, welche durch ein Verfahren hergestellt ist, welches ein separates Pressen des Teiles aus porösem Keramikmaterial (4) und des Teiles aus dichtem Keramikmaterial (5), optional Kalzionieren dieser Teile, Ineinandersetzen und Kombinieren dieser Teile sowie Sintern einer Kombination dieser Teile umfaßt, um eine integrale Struktur der genannten Teile durch Schrumpfverbindung dieser Teile zu erhalten.

8. Transkutanvorrichtung nach Anspruch 7, dadurch gekennzeichnet, daß das Sintern bei einer Temperatur von 1000 bis 1250 °C durchgeführt wird, wobei sowohl das verwendete poröse (4) als auch das verwendete dichte (5) Keramikmaterial Kalziumphosphat-Keramik sind.

9. Transkutanvorrichtung nach Anspruch 7, wobei das Sintern bei einer Temperatur von 1400 bis 1600 °C durchgeführt wird, wobei sowohl das verwendete poröse (4) als auch das verwendete dichte (5) Keramikmaterial Zirkonkeramik sind.

10. Transkutanvorrichtung nach Anspruch 7, wobei das Sintern bei einer Temperatur von 1200 bis 1400 °C durchgeführt wird, wobei das verwendete poröse Keramikmaterial (4) Hydroxylapatit ist und das verwendete dichte Keramikmaterial (5) Aluminiumoxid ist.

11. Transkutanvorrichtung nach irgendeinem der Ansprüche 1 bis 10, welche verwendet wird, um einen Katheter im Körper des Patienten festzuhalten.

## Revendications

1. Dispositif transcutané (1) permettant de retenir un équipement médical dans le corps d'un patient,. présentant un perçage(2) recevant l'équipement médical et comprenant une partie (4) conçue pour être implantée dans un tissu subcutané (3) du patient, partie qui est constituée d'un matériau céramique poreux, caractérisé en ce que les parties (5) autres que la partie venant en contact avec le tissu subcutané (3) sont constituées d'un matériau céramique dense.

2. Dispositif transcutané selon la revendication 1, dans lequel la céramique poreuse est choisie dans le groupe constitué des céramiques de phosphates de calcium, des céramiques d'alumines, des céramiques de zircones et leur composites.

3. Dispositif transcutané selon la revendication 2, dans lequel la céramique poreuse est de l'kydroxyapatite.

4. Dispositif transcutané selon la revendication 1, dans lequel le matériau céramique poreux a une porosité de 20 à 70% et des dimensions de pores de 2 à 2000 micromètres.

5. Dispositif transcutané selon la revendication 1, dans lequel la céramique dense est de même nature ou d'une nature différente que la matériau céramique poreux.

6. Dispositif transcutané selon la revendication 5, dans lequel à la fois les matériaux céramiques poreux et dense utilisés sont de l'hydroxyapatite.

7. Dispositif transcutané selon la revendication 1, préparé par un procédé comprenant les étapes consistant à mouler séparément la partie en matériau céramique poreux (4) et la partie en matériau céramique dense (5), à calciner s'il y lieu ces parties, à ajuster et combiner ces parties et à fritter la combinaison, pour former de ces parties une structure d'une seule pièce par ajustement par retraint de celles-ci.

8. Dispositif transcutané selon la revendication 7, dans lequel le frittage est conduit à une température de 1000 à 1250xC, quand à la fois les matériaux céramiques poreux (4) et dense (5) utilises sont des céramiques de calcium de phosphate.

9. Dispositif transcutané selon la revendication 7, dans lequel le frittage est conduit à une température de 1400 à 1600xC, quand à la fois les matériaux céramiques poreux (4) et dense (5) utilisés sont des céramiques de zircones.

10. Dispositif transcutané selon la revendication 7, dans lequel le frittage est conduit à une température de 1200 à 1400xC, quand le matériau céramique poreux (4) utilisé est de l'hydroxyapatite et le matériau céramique dense (5) utilisé est de l'alumine.

11. Dispositif transcutané selon l'une quelconque des revendications 1 à 10, quand il est utilisé pour retenir un cathéter dans le corps d'un patient.
